# EUROPEAN PATENT APPLICATION

(11) **EP 2 055 291 A1**
(43) Date of publication of application: **06.05.2009**
(21) Application number: 08168222.1
(22) Date of filing: 03.11.2008
(51) Int. Cl.: A61J 1/20, A61M 5/31

(54) **Device designed to aid drawing of liquid medicaments from containers**

(30) Priority: 02.11.2007 IT TO20070778
(71) Applicant: Policare S.r.l., 10078 Venaria Reale (IT)
(72) Inventor: Forino, Luciano, 10100 Torino (IT)
(74) Representative: Jorio, Paolo

(57) **Abstract**

Described herein is a device (1) designed to aid drawing of a liquid medicament from a container (2), provided with: attachment means (5) designed to be fixed in a releasable way to an inlet mouth (6) of a syringe (3); means (7) for immersion in the container (2), which define with the attachment means (5) a channel (8) for passage of the medicament; and filtering means (10) set along the channel (8) for withholding any possible particulate contaminants present in said medicament.

## Description

The present invention relates to a device designed to aid drawing of liquid medicaments from containers, such as, for example, sealed phials or flasks, to which the ensuing description will make explicit reference, without this implying any loss of generality.

It is pointed out that in the ensuing description and in the annexed claims, the term "medicament" is used to indicate any substance for medical use, such as for example curative, therapeutic, or diagnostic substances, including anaesthetic substances.

As is known, many liquid medicaments that are to be injected into the body of a patient are typically provided in phials or other types of containers that must meet extremely stringent standards of production to be suitable for pharmaceutical uses.

In particular, the aforesaid containers must be able to ensure protection of the contents without interacting physically or chemically with said contents, thus altering quality thereof beyond the limits tolerated by official specifications.

In the case of phials, which, as is known, are typically thinwalled glass or plastic containers, which are heat-sealed after being filled, an important parameter to be taken into consideration for the purposes of chemical stability is the hydrolytic resistance, i.e., the resistance to yielding to the water soluble mineral substances in precise conditions of contact between the water and the inner surface of the container.

The present applicant has noted that, notwithstanding the extremely stringent standards that regulate the types of materials for containers for pharmaceutical use in order to prevent the possible occurrence of alterations, beyond certain limits, of the medicaments contained therein, an important factor of risk of alteration derives, however, from the typical modalities with which the aforesaid medicaments are drawn from the corresponding containers to be injected into the patients.

In particular, drawing of the medicament normally entails breaking of the phial in a predetermined section and insertion of the needle of the syringe in the residual bottom part of the phial itself.

The present applicant has noted that, during the operation of breaking of the phial, some fragments of microscopic dimensions (which have been found, for example, to have dimensions of between a few microns and 100 - 150 µm) can drop into the medicament drawn from the syringe, thus contaminating it.

The present applicant has moreover noted that also other types of particulate contaminants can be drawn from the syringes during suction of the medicament from the corresponding container; amongst these, the most common are:
- milling fragments released by the needles of the syringes and deriving from the processes of production of the needles themselves;
- particles of latex or talcum deriving from the sterile gloves worn by the operator;
- fibres of fabric, deriving, for example, from gauze, cotton, garments, etc.; and
- hairs and dandruff, environmental dust, and microorganisms.

It is evident how suction of medicaments containing the aforementioned particulate contaminants can lead to complications in the treatment of patients basically according to the site in which the injection is made and to the nature and size of the aforesaid contaminants. In any case, the presence of said contaminants leads to a loss of sterility of the medicament to be administered.

In particular, in the case of administration of anaesthetics for intravenous use, the presence of particulate contaminants can play an important role in the formation of thrombi and/or granulomas, in the destruction of the vascular endothelium, in the onset of respiratory insufficiency, etc.; as regards, instead, the administration of anaesthetics for peridural or subarachnoidal use, the presence of particulate contaminants can have extremely important consequences including even neural or neuropathic ones.

The aim of the present invention is to provide a device designed to aid drawing of liquid medicaments from containers, which will enable the problem specified above to be overcome in a simple and inexpensive way.

The aforesaid purpose is achieved by the present invention in so far as it relates to a device designed to aid drawing of a liquid medicament from a container, characterized in that it comprises attachment means designed to be fixed in a releasable way to an inlet mouth of a syringe, means for immersion in said container, which define with said attachment means a channel for passage of said medicament, and filtering means set along said channel for withholding any possible particulate contaminants present in said medicament.

For a better understanding of the present invention, described in what follows is a preferred embodiment, which is provided purely by way of non-limiting example and with reference to the attached drawings, wherein:
- Figure 1 illustrates, in side view, a device according to the present invention applied on a corresponding syringe and while drawing a liquid medicament from a corresponding container;
- Figure 2 illustrates, in side view, the device and the syringe of Figure 1 after drawing of the medicament from the container;
- Figure 3 illustrates, in side view at an enlarged scale, the device of Figure 1 applied on the corresponding syringe; and
- Figure 4 is a cross section according to the line IV-IV of Figure 3.

With reference to the attached figures, designated as a whole by 1 is a device designed to aid drawing of liquid medicaments from containers 2, for example, from phials subjected to breaking to gain access to the medicaments themselves.

More precisely, the device 1 is of the type suited to being mounted on a syringe 3 instead of the corresponding needle 4, and usable exclusively for the operation of drawing of the medicament from the container 2.

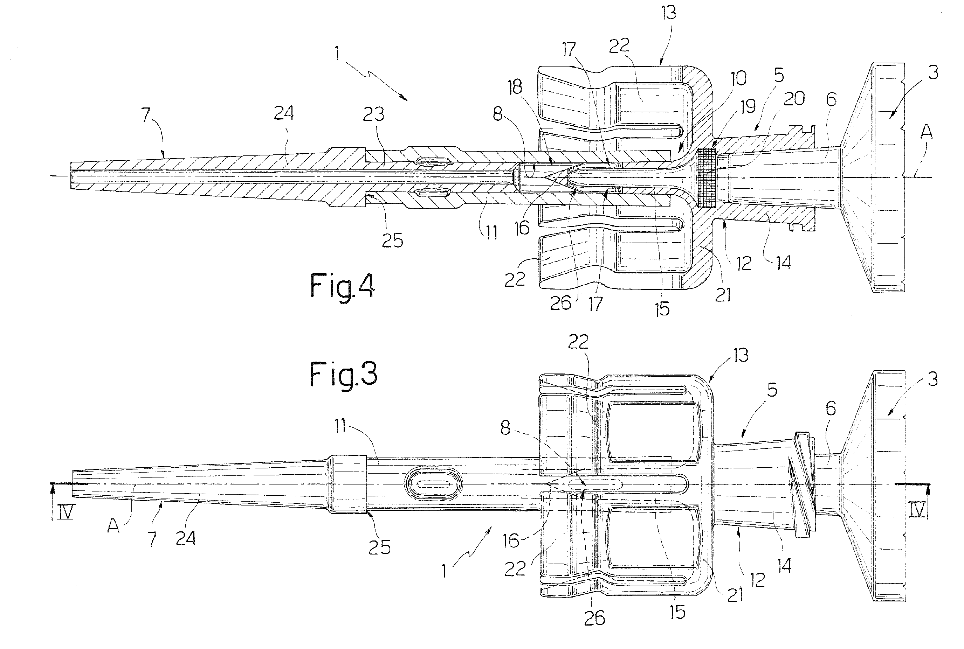

With particular reference to Figures 3 and 4, the device 1 has an axis A and basically comprises a hollow attachment element 5, designed to be fixed in a releasable way to an inlet mouth 6 of the syringe 3, an immersion element 7, which is also hollow, is designed to be introduced in use in the corresponding container 2, and defines, with the attachment element 5, a channel 8 for passage of the medicament, and filtering means 10 set along the channel 8 for withholding any possible particulate contaminants present in the medicament to be drawn.

The device 1 moreover comprises a flexible tube 11, preferably made of PVC, which connects together the attachment element 5 and the immersion element 7 and delimits therewith the channel 8.

The attachment element 5 is formed integrally by a tubular body 12, which connects the inlet mouth 6 of the syringe 3 to the flexible tube 11, and by a radially outermost gripping member 13, designed to enable gripping by the operator without contact with the channel 8 for passage of the medicament.

The tubular body 12 comprises a first portion 14 shaped like a truncated cone, designed to be engaged in use by the inlet mouth 6 of the syringe 3 and having sections that increase towards the syringe 3 itself, and a basically cylindrical second portion 15, radiused at one end to the portion 14 and closed, on the opposite side, by a pointed but non-piercing end 16.

The portion 15 of the attachment element 5 is designed to be engaged in use within an end portion of the flexible tube 11 and has windows 17 for passage of the medicament. In this way, made between the pointed end 16 and the inner surface of the flexible tube 11 is a restriction 26 of the section for passage of the medicament along the channel 8 with consequent production of a first filtering stage 18 for filtering the medicament itself, designed to withhold the contaminants of larger dimensions.

Advantageously, a second filtering stage 19 is formed by a filter 20, which is housed within the tubular body 12 in the area of radiusing between the portions 14 and 15 and is designed to withhold particulate contaminants of micrometric dimensions.

Preferably, the filter 20 is made of cellulose acetate and has a porosity comprised between 0.5 µm and 5 µm.

The filtering stages 18 and 19 define, as a whole, the filtering means 10. The filtering stage 18 is set between the filtering stage 19 and the immersion element 7.

The gripping member 13 comprises an annular flange 21 extending integrally outwards from the area of radiusing between the portions 14 and 15 of the tubular body 12, and a plurality of fins 22 projecting in cantilever fashion from the periphery of the flange 21 in a direction parallel to the axis A and spaced at equal angular distances apart around the end portion of the flexible tube 11 engaged by the portion 15 of the tubular body 12.

The immersion element 7 has internally a cylindrical conformation and externally a first cylindrical stretch 23, designed to be engaged within a corresponding end portion of the flexible tube 11, and a second stretch 24 shaped like a truncated cone, which has sections that decrease towards the free end starting from a cross section of dimensions larger than the stretch 23 and forming with the latter an annular contrast shoulder 25 for the flexible tube 11 itself.

The conformation of the immersion element 7, which is externally tapered towards its own free end, has the function of facilitating introduction of the immersion element 7 itself into phials of small dimensions, such as, for example, ones containing morphine.

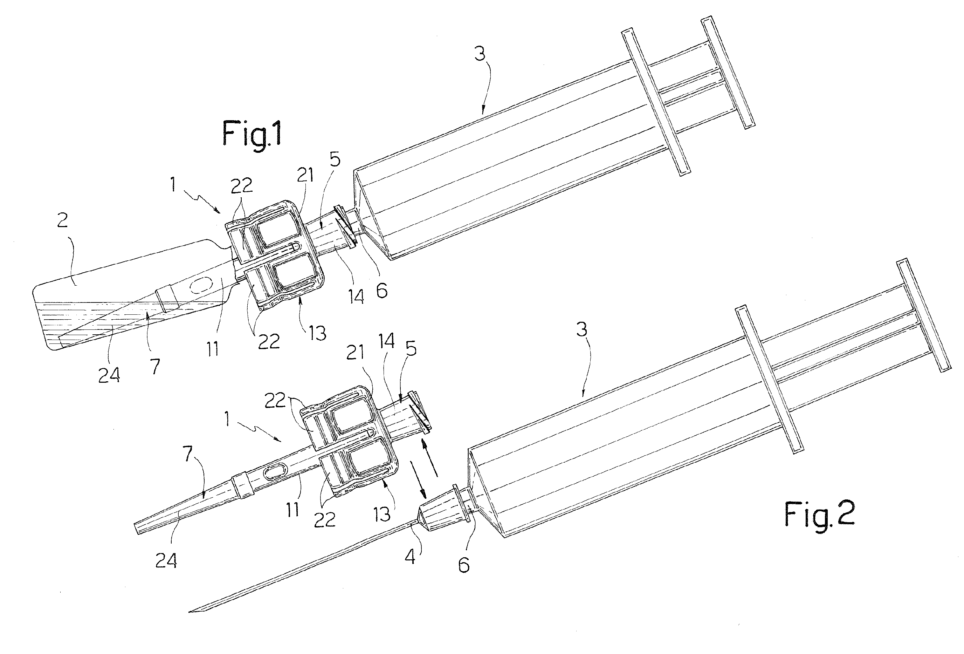

In use, the device 1 is connected to a corresponding syringe 3 by means of insertion of the inlet mouth 6 of the syringe 3 itself within the portion 14 shaped like a truncated cone of the tubular body 12 of the attachment element 5 (Figure 1). In this configuration, after the container 2 from which to draw the medicament has been broken, the immersion element 7 of the device 1 is introduced into the residual portion of the container 2 itself. The presence of the flexible tube 11 facilitates this operation.

At this point, by acting as is customary on the syringe 3, suction of the medicament is obtained, which, as it passes through the channel 8, traverses the filtering stages 18 and 19, with consequent removal of any possible particulate contaminants and elimination of the connected risks of subsequent complications in the patient, to whom the medicament is then administered.

At the end of this operation, the device 1 can be removed from the syringe 3, and the latter can be coupled with a corresponding needle 4 (Figure 2) to proceed with the administration referred to.

Finally, it is clear that modifications and variations can be made to the device 1 described and illustrated herein, without thereby departing from the sphere of protection defined by the claims.

## Claims

1. A device (1) designed to aid drawing of a liquid medicament from a container (2), **characterized in that** it comprises attachment means (5) designed to be fixed in a releasable way to an inlet mouth (6) of a syringe (3), means (7) for immersion in said container (2), which define with said attachment means (5) a channel (8) for passage of said medicament, and filtering means (10) set along said channel (8) for holding back any possible particulate contaminants present in said medicament.

2. The device according to Claim 1, **characterized in that** said filtering means (10) comprise a filter (20) designed to withhold particulate contaminants of micrometric dimensions.

3. The device according to Claim 1, **characterized in that** said filter (20) has a porosity comprised between 0.5 µm and 5 µm.

4. The device according to Claim 2 or Claim 3, **characterized in that** said filtering means (10) comprise at least one restriction (26) of the cross section of passage of said channel (8), said restriction (26) being set between said filter (20) and said immersion means (7).

5. The device according to any one of the preceding claims, **characterized in that** said channel (8) is delimited by a portion made of flexible material (11), set between said attachment means (5) and said immersion means (7).

6. The device according to any one of the preceding claims, **characterized in that** it comprises gripping means (13) surrounding said channel (8) on the outside and designed to define a support for the hands of the user.

7. The device according to any one of the preceding claims, **characterized in that** said immersion means comprise a hollow portion (7) externally tapered towards the free end of introduction in said container (2) and non-piercing.
